(19) Europäisches Patentamt
European Patent Office
Office européen des brevets

(11) **EP 3 891 368 B1**

(12) **EUROPÄISCHE PATENTSCHRIFT**

(45) Veröffentlichungstag und Bekanntmachung des Hinweises auf die Patenterteilung:
**26.07.2023  Patentblatt 2023/30**

(21) Anmeldenummer: **19816530.0**

(22) Anmeldetag: **03.12.2019**

(51) Internationale Patentklassifikation (IPC):
*F01M 11/10* $^{(2006.01)}$   *F16N 29/04* $^{(2006.01)}$
*G01M 15/04* $^{(2006.01)}$   *G01M 15/10* $^{(2006.01)}$
*G01N 33/30* $^{(2006.01)}$   *G06N 3/08* $^{(2023.01)}$

(52) Gemeinsame Patentklassifikation (CPC):
**F01M 11/10; F16N 29/04; G01M 15/042;
G01M 15/102; G01N 33/30; G06N 3/08;**
F01M 2011/14; F16N 2200/00

(86) Internationale Anmeldenummer:
**PCT/EP2019/000331**

(87) Internationale Veröffentlichungsnummer:
**WO 2020/114622 (11.06.2020 Gazette 2020/24)**

(54) **VERFAHREN ZUR BESTIMMUNG UND PRÄDIKTION DES INDIVIDUELLEN ÖLWECHSELINTERVALLS EINES VERBRENNUNGSMOTORS**

METHOD FOR DETERMINING AND PREDICTING THE INDIVIDUAL OIL CHANGE INTERVAL OF AN INTERNAL COMBUSTION ENGINE

PROCÉDÉ PERMETTANT DE DÉTERMINER ET DE PRÉDIRE LA PÉRIODICITÉ INDIVIDUELLE DES VIDANGES D'UN MOTEUR À COMBUSTION INTERNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.12.2018   DE 102018009521**

(43) Veröffentlichungstag der Anmeldung:
**13.10.2021   Patentblatt 2021/41**

(73) Patentinhaber: **DEUTZ Aktiengesellschaft**
**51149 Köln (DE)**

(72) Erfinder:
• **STEINHAUSEN, Andreas**
**50859 Köln (DE)**
• **JOISTEN-PIERITZ, Joachim**
**53925 Kall-Steinfeld (DE)**
• **WINKLER, Markus**
**46242 Bottrop (DE)**
• **KLEIN, Ulf**
**53804 Much (DE)**
• **HOEN, Thomas**
**51503 Rösrath (DE)**

(56) Entgegenhaltungen:
DE-A1-102007 042 507    DE-A1-102014 013 709
US-A- 5 969 601    US-A1- 2010 299 080
US-A1- 2018 202 333

• EPA: "APPENDIX VI TO PART 1039-NONROAD COMPRESSION-IGNITION COMPOSITE TRANSIENT CYCLE", CODE OF FEDERAL REGULATIONS - TITLE 40. PROTECTION OF ENVIRONMENT, 29. Juni 2004 (2004-06-29), Seiten 159-167, XP055671297,

**Beschreibung**

Derzeit sind keine technischen Lösungen oder Umsetzungen zur Bestimmung individueller Ölwechselintervalle bekannt. Das aktuelle Verfahren definiert präventiv statische Ölwechselintervallzeiten, unabhängig von der individuellen Nutzung eines Verbrennungsmotors. Hierbei handelt es sich um eine Methode der Worst Case Abschätzung, basierend auf umfangreichen Versuchsreihen. Für bestimmte Baugruppen werden verschiedene Prüfstands-Messungen durchgeführt, dazu zählen auch Ölanalysen der entnommenen Ölproben. Die Motorbaugruppen werden mit repräsentativen Lastzyklen im Rahmen von Dauerläufen belastet. Anschließend wird das Öl analysiert. Das Ergebnis ist eine Worst Case Abschätzung der Öllebensdauer auf Basis der durchgeführten Dauerläufe. Hierbei wird stets ein Sicherheitsfaktor eingerechnet. Nachteilig daran ist, dass nicht jeder individuelle Verbrennungsmotor in der Praxis ähnlich ausgelastet wird, wie in den zu Grunde liegenden Dauerläufen angenommen. Darauf addiert sich unter Umständen der Sicherheitsfaktor. US2018/202333 A1 beschreibt ein Beispiel eines Verfahrens zur Vorhersage der verbleibenden Öllebensdauer. Aufgabe der vorliegenden Erfindung ist es, ein Verfahren zu schaffen, dass die Öllebensdauer für den jeweiligen Verbrennungsmotor, unter Vermeidung starrer Worst Case Intervalle, mit einer beliebigen individuellen Auslastung bzw. Nutzung bestimmen soll.

Die Aufgabe wird durch ein Verfahren nach Anspruch 1 gelöst. Weitere vorteilhafte Ausgestaltungen sind den Unteransprüchen zu entnehmen.

Die Bestimmung und Vorhersage der tatsächlich verbleibenden Ölbetriebsdauer eines Verbrennungsmotors erfolgt in Abhängigkeit der individuellen Nutzung des Verbrennungsmotors.

Die individuelle Ölbetriebsdauer kann deutlich länger ausfallen als ein zuvor statisch festgelegtes Ölwechselintervall, welches sich nicht auf die individuelle Nutzung des Verbrennungsmotors (in der Praxis) bezieht.

Durch das erfindungsgemäße Verfahren erfolgt eine Reduktion der Maschinen Downtime durch potentiell längere Ölbetriebsdauer. Eine Einsparung von Öl und eine Reduzierung von Betriebs- und Wartungskosten ist die Folge.

Der Ölzustand des Verbrennungsmotors ist bekannt. Die Kenntnis des Ölzustands ermöglicht eine Prognose über den Zeitpunkt, wann der nächste Ölwechsel optimalerweise durchzuführen ist (Predictive Maintenance).

Abgrenzung zu den typischen Verfahren: Bonus Malus, und mathematisches Modell

Das Bonus Malus Verfahren stellt eine Tabelle zur Verfügung, die die jeweiligen "Strafwerte" beinhaltet. Je nach Nutzung der Maschine wird ein Malus abgezogen (von der Öllebensdauer) bzw. ein Bonus addiert. Die "Strafwerte" werden durch eine heterogene Testflotte, welche Millionen von Kilometern zurückgelegt hat, erfasst. Die Daten der Testflotte werden anschließend auf jedwedes Fahrzeug generalisiert. Das zu patentierende Verfahren arbeitet nicht mit Bonus Malus Tabellen und auch nicht mit Testflotten Datenerhebung. Das hier beschriebene Verfahren arbeitet mit künstlichen neuronalen Netzen, die den jeweiligen Ölverschleiß in Abhängigkeit der individuellen Nutzung konkret und absolut berechnet. Die künstlichen neuronalen Netze werden durch heuristische Algorithmen optimiert und bilden ein mathematisches Modell des Verschleißprozesses, Bonus Malus Verfahren nutzen eine tabellarische Berechnungsvorschrift. Die Trainingsdaten werden im Gegensatz zu den heterogenen Testflottendaten an homogenen Motorbaureihen erhoben.

Ein weiteres gängiges Verfahren ist die Verwendung von mathematischen Modellen. Hierbei wird ein mathematisches Modell (z. B. Polynom, Gerade, etc.) für den Verschleißprozess angenommen. Die Koeffizienten des mathematischen Modells werden mittels Messdaten und Optimierungsverfahren an eine Anwendung angepasst. Im Unterschied zu den mathematischen Modellen verwendet das hier beschriebene Verfahren Machine Learning zur Bildung eines Degradationsmodells des Verschleißprozesses. Das Degradationsmodell besteht aus mehreren künstlichen neuronalen Netzen, die je ein Verschleißverhalten eines Schadensindikators prädizieren. Ein mathematisches Modell besteht typischerweise aus einer Funktion, z. B. Polynom, welches auf ein beliebiges Degradationsverhalten gefittet (angepasst) wird. Die Basis bleibt jedoch immer ein Polynom, auch wenn das Verhalten der Degradation ein anderes ist (die Verschleißindikatoren können unterschiedliche Verschleißverhalten haben z. B. polynomial, exponentiell, linear, etc.). Die künstlichen neuronalen Netze haben den Vorteil, dass sie Universal-Approximatoren sind. D. h. sie können beliebige Funktionen annehmen und sich somit individuell an das Verschleißverhalten anpassen.

Ein weiterer maßgebender Unterschied zu vielen patentierten Verfahren ist, dass das hier beschriebene Verfahren gänzlich ohne Ölqualitätssensoren auskommt. Es werden demnach keine zusätzlichen Sensoren zur Bestimmung des Ölzustands benötigt. Abgesehen von der Öltemperatur werden indirekte, im Motorsteuergerät berechnete Motorsignale als Grundlage zur Bestimmung des Ölzustands verwendet.

Weitere wichtige Merkmale und Vorteile ergeben sich aus den Unteransprüchen, aus den Zeichnungen und aus der nachfolgenden Beschreibung eines bevorzugten Ausführungsbeispiels anhand der Zeichnungen. Es zeigen:

Fig. 1    Nonroad Transient Cycle Zusammensetzung durch Anwendungsprofile [Quelle: Control of Emissions from Nonroad Diesel Engines, EPA-420-R-03-008, April 2003, www.epa.gov]

Fig. 2    Bestandteile und Verfahren der prädiktiven Bestimmung des individuellen Ölwechselintervalls

Fig. 3    Schwellwerteüberwachung und Berechnung des individuellen Öl-wechselintervalls.

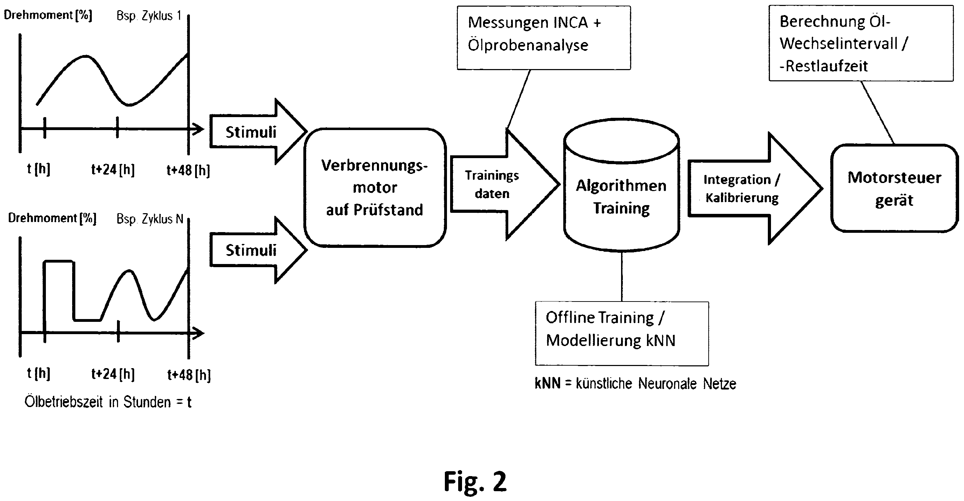

Das Verfahren setzt sich aus den folgenden Bestandteilen zusammen, wie dies in Fig. 2 dargestellt wird und wobei Verfahren zur Bestimmung und Prädiktion des individuellen Ölwechselintervalls eines Verbrennungsmotors die nachfolgenden Schritte umfasst:

- Ermittlung und Speicherung der Einflussgrößen und Verschleißindikatoren des Öls, die einen Ölverschleiß hervorrufen können an einem Verbrennungsmotor auf dem Prüfstand, insbesondere unter Berücksichtigung der aus dem Nonroad Transient Cycle (NRTC) extrahierten Lastzyklen, Messungen der Lastzyklen am Prüfstand, Vornahme von Ölanalysen, Modellbildung und Implementierung in das Motorsteuergerät, Zeitreihenanalyse zur Prädiktion des individuellen Ölwechselintervalls im laufenden Betrieb des Verbrennungsmotors
- Anzeige des vorhergesagten Ölwechselzeitpunkts, verbleibende Öllebensdauer in Betriebsstunden
- Einflussgrößen, die Ölverschleiß hervorrufen können
- Verschleißindikatoren des Öls
- Lastzyklen, extrahiert aus dem Nonroad Transient Cycle (NRTC)
- Verbrennungsmotor
- Messungen der Lastzyklen am Prüfstand
- Ölanalysen
- Trainingsdaten
- Methoden aus dem Bereich Machine Learning und künstliche neuronale Netze
- Motorsteuergerät
- Zeitreihenanalyse zur Prädiktion des individuellen Ölwechselintervalls.

Die Einflussgrößen, die den Ölzustand verändern, sind die integrale Öltemperatur (z. B. in der Ölwanne) über die Laufzeit des Verbrennungsmotors und/oder die Rußemission durch den Verbrennungsprozess und/oder die NOx-Emission, die durch Verbrennungsprozess entsteht und/oder die Drehzahl (Integral oder Mittelwert) und/oder das Drehmoment (Integral oder Mittelwert) und/oder die mittlere Leistung und/oder die Anzahl von Kaltstarts und die Verweildauer im Kaltbetrieb und /oder die Kraftstoff und Ölqualität.

Die Ölanalyse stellt Veränderungen des Ölzustands anhand von Verschleißindikatoren fest. Die wichtigsten Verschleißindikatoren sind u. a. die Folgenden:

1. Rußgehalt im Öl
2. Gesamtsäurezahl / Gesamtbasenzahl
3. Viskosität
4. Verschiedene Fremdstoffe
5. Kraftstoff im Öl
6. Wassergehalt im Öl.

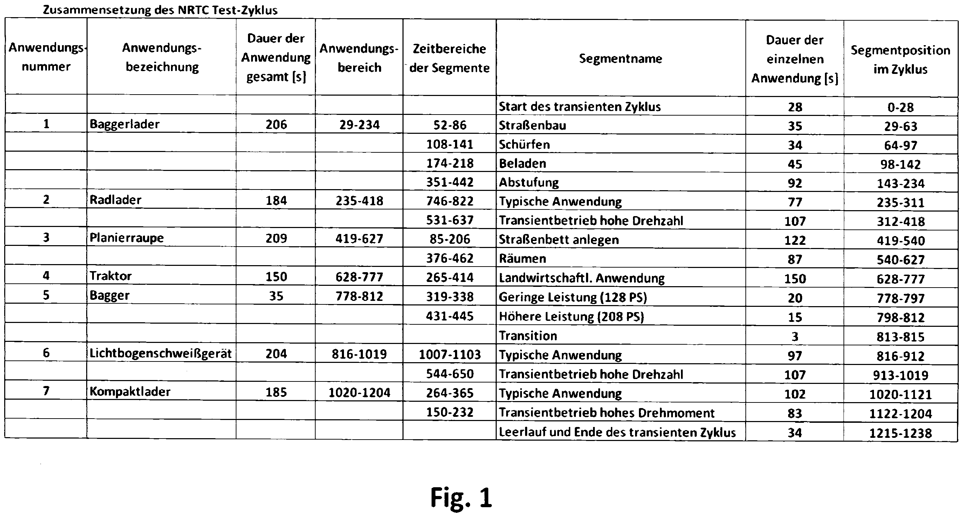

Zuerst müssen bestimmte anwendungsnahe Lastzyklen definiert werden. Hierfür wird u. a. der standardisierte Nonroad Transient Cycle (NRTC) verwendet, wie dies in Fig. 1 offenbart wird. Besonders wichtig bei der Definition der Lastzyklen ist: Die Lastzyklen sollten möglichst realistischen und praxisnahen Anwendungen entsprechen, damit das realistische Verhalten durch das Training der Algorithmen im Motorsteuergerät modelliert werden kann. Anwendungsnah bedeutet, dass die typischen Anwendungsprofile wie z. B. Baggerlader, Radlader, Traktor, etc. in den verschiedenen Lastzyklen enthalten sind. Der NRTC Zyklus setzt sich, wie in Fig. 1 gezeigt, aus verschiedenen Anwendungsprofilen zusammen.

Der NRTC enthält sieben verschiedene anwendungsnahe Nutzungsprofile. Diese sieben Profile werden im nächsten Schritt isoliert. Damit entstehen sieben verschiedene Lastzyklen, welche dazu benutzt werden, die Trainingsdaten zu produzieren.

Ziel ist, die sieben Anwendungszyklen am Prüfstand zu reproduzieren und den Einfluss der verschiedenen Zyklen auf den Ölzustand zu identifizieren. Die Änderung des Ölzustands ist zeitabhängig. Die einzelnen Zykluszeiten betragen nur wenige Minuten. Innerhalb weniger Minuten Betriebszeit wird sich der Ölzustand nicht messbar verändern können. Aufgrund dessen werden die Zyklen im nächsten Schritt zeitlich skaliert. Die isolierten Zyklen werden jeweils hintereinander reproduziert bis eine Zykluszeit von z. B. 24 h entsteht. Nach 24 h wird die erste Ölprobe entnommen. Dabei ist zu beachten, dass die Entnahmemenge möglichst klein gehalten werden sollte, damit die Messung durch Nachfüllen von "Frischöl" nicht zu stark verfälscht wird. Nun wird der Zyklus weitere 24 h wiederholt. Eine weitere Ölprobe wird nach 48 h entnommen. Je öfter man dies wiederholt, desto größer ist der Stichprobenumfang.

Zwischen den sieben verschiedenen Zyklen müssen jedes Mal einmalig vor Beginn der nächsten Zyklusmessung ein Ölwechsel und eine Referenzölprobenentnahme erfolgen. Der Ölzustand (die Verschleißindikatoren des Öls, z. B. Ruß) erfährt innerhalb der jeweiligen Zyklusmessungen eine lineare Verschlechterung. Grundsätzlich ist der Ölverschleiß nicht linear, aber dadurch, dass die Eingangsgrößen (Lastzyklen) über die Zeit gleich bleiben, erfährt das Öl eine lineare Verschlechterung. Wichtig ist, dass dieses lineare Verhalten (mittlere Steigung) in allen 24 h Intervallen (bis auf Messungenauigkeiten) tendenziell identisch bleibt. Falls dies nicht der Fall ist, hat sich an den Rahmenbedingungen der Messung eine Veränderung ergeben, die Auswirkunken auf die Messung hat. In diesem Fall muss die Messung wiederholt werden. Um darüber hinaus Veränderungen an der Messmetrik oder am Verbrennungsmotorzustand zu entdecken, empfiehlt es sich, regelmäßig Referenzmessungen (z. B. 5 h Volllastfahrt) durchzuführen. Damit sind Veränderungen des Motorzustands oder der Messmetrik erkennbar. Die Referenzmessungen dürfen nicht während einer Zyklusmessung stattfinden, sondern ausschließlich zwischen den verschiedenen Zyklusmessungen.

Grundsätzlich ist darauf zu achten, dass die Varianz des Eingangsraums (Varianz der verschiedenen Lastzyklen) möglichst hoch ist. Wenn ausschließlich Zyklen mit hoher oder niedriger Auslastung verwendet werden, steigt die Auftretenswahrscheinlichkeit der Messungen an und die (Informationstechnik) Entropie sinkt. Das hat zu Folge, dass nicht genügend Informationsdichte für das Training der künstlichen neuronalen Netze vorhanden ist. Zusätzlich müssen die künstlichen neuronalen Netze in gering trainierten Bereichen stark extrapolieren, was dazu führen kann, dass die Modellgüte in diesen Bereichen deutlich schlechter ist. Je nach Anwendung des Verbrennungsmotors kann es notwendig sein, weitere Nutzungsprofile, wie bspw. einen Gabelstapler, ein Genset oder einen Kaltstartzyklus zu ergänzen. Damit erhöht man die Generalisierbarkeit der Algorithmen und damit die Genauigkeit der Ölwechselintervalle für alle Anwendungen.

Während der Vermessung am Prüfstand werden die Einflussgrößen aufgezeichnet und gespeichert. Die Ölproben werden entnommen und analysiert. Die Trainingsdaten setzen sich aus den gemessenen integralen Einflussgrößen über den jeweiligen Zykluszeitraum und dem Verlauf der Verschleißindikatoren des Öls zusammen.

Bsp.: Bei einem mittleren Drehmoment von 80 % über 24 h steigt der Rußgehalt im Öl um 0,015 % an und die Viskosität steigt proportional an.

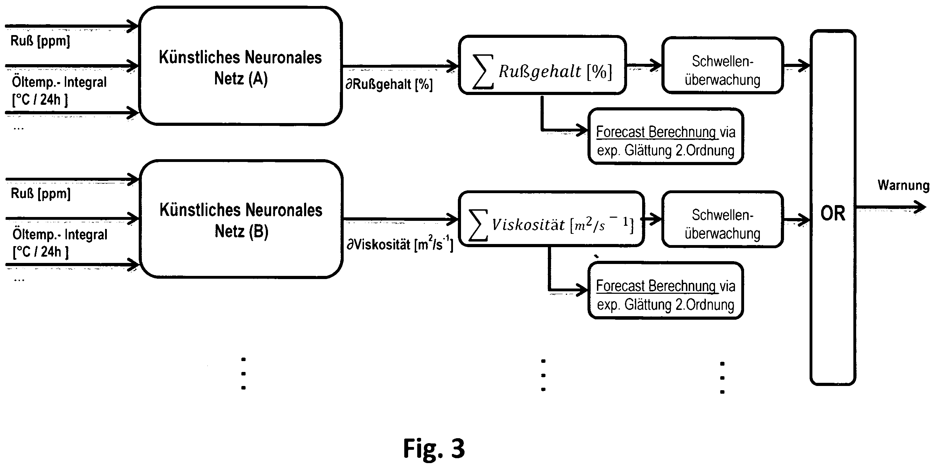

Alle Einflussgrößen werden als Integral oder als Mittelwert erfasst und zum Eingangsvektor des künstlichen neuronalen Netzwerks zusammengefasst. Zielgröße ist der jeweilige gemessene (Ölanalyse) Schadensindikator (Fig. 3).

Vorhersage des Ölwechselintervalls:

Nachdem die künstlichen neuronalen Netze trainiert sind, werden ihre Parameter in das Motorsteuergerät kalibriert. Das Motorsteuergerät misst zur Betriebszeit des Verbrennungsmotors die Einflussgrößen, integriert diese oder bildet Mittelwerte je Beobachtungszeitraum (hier 24 h). D. h. alle 24 h werden die Einflussgrößen durch die künstlichen neuronalen Netze verarbeitet und der Schadensindikator neu berechnet. Der neue Schadensindikator der letzten 24 h wird jeweils auf die Gesamtschädigung addiert. Die Gesamtschädigungen aller Schadensindikatoren bilden den globalen Ölzustand.

Die akkumulierten Schadensindikatoren werden nach jedem Beobachtungsintervall mit absoluten Schwellwerten verglichen. Je nach Konfiguration wird eine Warnung generiert, die signalisiert, dass ein Verschleißindikator die Grenzwerte überschritten hat und damit das Öl verschlissen ist. Nun sollte umgehend ein Ölwechsel stattfinden.

Für den Forecast / die Prognose des individuellen Ölwechselintervalls wird das Verfahren der Zeitreihenanalyse in Kombination mit dem Verfahren der exponentiellen Glättung verwendet. Hierfür wird ein lineares Trendmodell zur stückweisen linearen Approximation des Prozesses (Ölverschleiß / -alterung) verwendet. Durch Unterabtastung (Abtastung im Stundenbereich statt in Sekunden) wird der Prozess stark geglättet, was sich vorteilhaft auf die lineare Approximation auswirkt. Mit dem Forecast kann prognostiziert werden, wie hoch der Schadensindikator nach dem nächsten Forecast Horizont (z. B. 100 Betriebsstunden) sein wird. Der Forecast wird für je einen akkumulierten Schadensindikator berechnet (Fig. 3).

Berechnung 1. Ordnung als Zwischenwert:

$Z1_t$ [%]     = Zwischenwert der exponentiellen Glättung 1. Ordnung
$\alpha$     = Glättungskonstante für exponentiellen Glättung 1. Ordnung
$o_{bsrv}$ [%]     = aktueller Wert des jeweiligen Schadensindikators
$Z1_{t-1}$ [%]     = vorheriger Zwischenwert

$$Z1_t = \alpha \cdot O_{bsrv} + (1 - \alpha) \cdot Z1_{t-1} \qquad (1)$$

Berechnung 2. Ordnung:

$Z2_t$ [%] = Zwischenwert der exponentiellen Glättung 2. Ordnung
$\beta$ = Glättungskonstante für exponentiellen Glättung 2. Ordnung
$Z1_t$ [%] = Zwischenwert der exponentiellen Glättung 1. Ordnung
$Z2_{t-1}$ [%] = vorheriger Zwischenwert

$$Z2_t = \beta \cdot Z1_t + (1 - \beta) \cdot Z2_{t-1} \qquad (2)$$

Die Glättungskonstanten ($\alpha$ und $\beta$) können mittels Felddaten und Simulation des Prozesses oder mit Hilfe eines Optimierungsverfahrens ermittelt werden.

Nachdem die exponentielle Glättung berechnet wurde (für den gesamten Forecast Horizont), wird Formel (3) angewandt. Die aktuellen Glättungsfaktoren zum Zeitpunkt des Forecast Horizonts werden wiederum geglättet und mit dem Forecast Horizont multipliziert. Dazu wird der Y-Achsenabschnitt addiert und das Ergebnis ist der Forecast (= verbleibende Ölbetriebszeit nach FCH) zum Zeitpunkt FCH.

$$Forecast_{FCH} = (2 \cdot Z1_{t+FCH}) - Z2_{t+FCH} + FCH \cdot \alpha \cdot (Z1_{t+FCH} - Z2_{t+FCH}) \qquad (3)$$

Berechnung des Ölwechselintervalls:

Durch Aufstellung der Geradengleichung und Einsetzen der Schadensindikatorgrenze (Schwelle) in die Gleichung kann der Zeitpunkt, wann das Öl verschlissen sein wird, bestimmt werden. Dies gelingt durch Anwendung der Formeln 4 bis 6. Es werden die Steigung m (mittlere Steigung) über den gesamten Forecast Horizont und der Y-Achsenabschnitt berechnet. Dann wird die Geradengleichung nach der (Rest-) Betriebszeit aufgelöst. $o_{bsrvMax}$ entspricht in diesem Fall der jeweiligen Schadensschwelle. Die Berechnung wird für jeden einzelnen Schadensindikator durchgeführt. Nun wird die geringste Betriebslaufzeit von allen Schadensindikatoren ermittelt und als restliche Betriebslaufzeit des Öls angenommen. Diese stellt den nächsten Ölwechselzeitpunkt in Betriebsstunden (Ölwechselintervall) dar.

$Forecast_{time}$ [h] = Betriebszeit in Stunden bis Erreichen $o_{bsrvMax}$
$o_{bsrvMax}$[%] = obere Schwelle des Verschleißes, hier: 100% Ölverschleiß
$m$ [%/FCH] = Steigung
$b$ [%] = Y-Achsenabschnitt
$Z1_{t+FCH}$[%] = Zwischenwert der exponentiellen Glättung 1. Ordnung zum Zeitpunkt $_{FCH}$
$FCH$[h] = Forecast Horizont (z. B. 24 h $\cdot$ 2 (Beobachtungsintervall) = 48 $h$ = FCH
$Z2_{t+FCH}$[%] = Zwischenwert der esponentiellen Glättung 2. Ordnung zum Zeitpunkt $_{FCH}$
$Forecast_{vcc}$[%] = Forecast Vektor der Länge $_{FCH}$
$T_{observ}$[h] = Beobachtungsintervall, eine Forecast Berechnung je $T_{observ}$
$x$ [h] = Restbetriebszeit

Geradengleichung:

$$Forecast_{time} = x = \left| \frac{(o_{bsrvMax} - b)}{m} \right| \qquad (4)$$

5

Mit:

Y-Achsenabschnitt $b$:  (5)

$$b = (2 \cdot Z1_{t+FCH}) - Z2_{t+FCH}$$

Steigung $m$:

$$m = \frac{Forecast_{vec}\,[end] - b}{T_{observ} \cdot length(Forecast_{vec})}$$  (6)

Berechnung Forecast Error:

Das Vorhersageergebnis wird während der Laufzeit permanent überwacht. Dazu wird der Prognosefehler (MAPE, Mean Absolut Percentage Error, siehe Formel 7) periodisch bestimmt. Zeichnet sich eine zu hohe Abweichung über mehrere Perioden ab, werden die Glättungskoeffizienten der exponentiellen Glättung stärker angepasst (Überschreiten = Verringerung der Glättungsfaktoren, Unterschreiten = Vergrößerung der Glättungskonstanten).

M [%]  = MAPE

$n$  = Anzahl der Prognosen

$A_t$ [%]  = aktueller Wert der mittleren rel. Abweichung

$F_t$ [%]  = Prognostizierter Wert der mittleren rel. Abweichung

$$M = \frac{100}{n} \sum_{t=1}^{n} \left| \frac{A_t - F_t}{A_t} \right|$$  (7)

Die Prognose des Ölwechselintervalls wird mit dem Regelwartungsintervall des Verbrennungsmotors abgeglichen. Kann ein Regelwartungsintervall zeitlich nicht erreicht werden, da die verbleibende Ölbetriebszeit geringer ist als die Differenz aus Zeitpunkt des Regelwartungsintervalls und verbleibende Betriebszeit, wird der einzuhaltende Zeitpunkt der Wartung aufgrund des berechneten Ölwechselintervalls kommuniziert bzw. gespeichert. Kann das Regelwartungsintervall zeitlich erreicht werden, wird der Status kommuniziert, dass das Öl bei der nächsten Regelwartung getauscht werden sollte.
Wird kein rechtzeitiger Ölwechsel durchgeführt, wird aufgrund der o. g. Warnung ein Diagnosestatus aktiviert.

Berechnung Korrekturfaktor:

Die Qualität des Öls, des Kraftstoffs und die Ölmenge werden jeweils durch einen Korrekturfaktor berücksichtigt. Nach Berechnung der jeweiligen Einzelschädigung durch die künstlichen neuronalen Netze wird in Abhängigkeit der Ölmenge, der Kraftstoffqualität und Ölqualität, ein anteiliger kalibrierbarer Offset addiert. Damit wird die Schadensschwelle der Schadensindikatoren, abhängig von der Öl-, Kraftstoffqualität und der Ölmenge, schneller erreicht.

**Patentansprüche**

**1.** Verfahren zur Bestimmung und Prädiktion eines individuellen Ölwechselintervalls eines Verbrennungsmotors, umfassend die nachfolgenden Schritte:

Ermittlung von Einflussgrößen, die einen Ölverschleiß hervorrufen können, und von Verschleißindikatoren des Öls an einem Verbrennungsmotor auf einem Prüfstand, wobei Messungen von Lastzyklen an dem Prüfstand durchgeführt werden und Ölanalysen vorgenommen werden,
Speicherung der gemessenen Einflussgrößen und Verschleißindikatoren,
Erstellen eines Modells aus den gemessenen Einflussgrößen und Verschleißindikatoren,
Implementierung des Modells in ein Motorsteuergerät,
Anzeige eines vorhergesagten Ölwechselzeitpunkts und einer verbleibenden Öllebensdauer in Betriebsstunden,
**dadurch gekennzeichnet, dass** eine Zeitreihenanalyse von Ausgabewerten des Modells zur Prädiktion des individuellen Ölwechselintervalls im laufenden Betrieb des Verbrennungsmotors durchgeführt wird.

2. Verfahren nach Anspruch 1,
**dadurch gekennzeichnet, dass** die Einflussgrößen, die den Ölzustand verändern, die folgenden umfassen:
eine integrale Öltemperatur über die Laufzeit des Verbrennungsmotors, eine mittlere Drehmomentänderung, eine mittlere Drehzahländerung, eine Rußemission durch den Verbrennungsprozess, eine NOx Emission, die durch Verbrennungsprozess entsteht, eine Drehzahl, ein Drehmoment, eine mittlere Leistung, eine Anzahl von Kaltstarts und eine Verweildauer im Kaltbetrieb, eine Kraftstoff- und Ölqualität,

3. Verfahren nach einem oder mehreren der vorgenannten Ansprüche,
**dadurch gekennzeichnet, dass** die Verschleißindikatoren die folgenden umfassen: ein Rußgehalt im Öl, eine Gesamtsäurenzahl / Gesamtbasenzahl, eine Viskosität, Fremdstoffe im Öl, Kraftstoff im Öl, ein Wassergehalt im Öl.

4. Brennkraftmaschine, **dadurch gekennzeichnet, dass** ein Verfahren nach einem oder mehreren der vorgenannten Ansprüche zum Einsatz kommt.

**Claims**

1. A method for determining and predicting an individual oil change interval of an internal combustion engine, comprising the following steps:

determining influencing variables, which can induce oil wear, and wear indicators of the oil on an internal combustion engine on a test stand, wherein measurements of load cycles are carried out on the test stand and oil analyses are performed,
storing the measured influencing variables and wear indicators,
creating a model from the measured influencing variables and wear indicators,
implementing the model in an engine control unit,
displaying a predicted oil change point in time and a remaining oil lifetime in operating hours,
**characterized in that** the time series analysis of output values of the model to predict the individual oil change interval is carried out in the running operation of the internal combustion engine.

2. The method according to Claim 1,
**characterized in that** the influencing variables which change the oil status comprise the following:

an integral oil temperature over the running time of the internal combustion engine, a mean toward change, a mean speed change, a soot emission due to the combustion process, a NOx emission which results due to the combustion process, a speed, a torque,
a mean power, a number of cold starts and a dwell time in called operation, a fuel quality and an oil quality.

3. The method according to one or more of the preceding claims,
**characterized in that** the wear indicators comprise the following: a soot content in the oil, a total acid number/total base number, a viscosity, foreign materials in the oil, fuel in the oil, a water content in oil.

4. An internal combustion engine, **characterized in that** a method according to one or more of the preceding claims is used.

**Revendications**

1. Procédé permettant de déterminer et de prédire un intervalle de vidange d'huile individuel d'un moteur thermique, comprenant les étapes suivantes consistant à :

   établir des grandeurs d'influence qui peuvent provoquer une usure de l'huile et des indicateurs d'usure de l'huile sur un moteur thermique sur un banc d'essai, dans lequel des mesures de cycles de charge sont effectuées sur le banc d'essai et des analyses d'huile sont effectuées,
   mémoriser les grandeurs d'influence et les indicateurs d'usure mesurés,
   créer un modèle à partir des grandeurs d'influence et des indicateurs d'usure mesurés,
   mettre en oeuvre le modèle dans un appareil de commande de moteur,
   afficher une date de vidange d'huile prédite et une durée de vie restante de l'huile en heures de fonctionnement, **caractérisé en ce qu'**une analyse de série chronologique de valeurs de sortie du modèle est effectuée pour prédire l'intervalle de vidange d'huile individuel en cours de fonctionnement du moteur thermique.

2. Procédé selon la revendication 1, **caractérisé en ce que** les grandeurs d'influence qui modifient l'état de l'huile comprennent les suivantes :
   une température d'huile intégrale pendant le temps de fonctionnement du moteur thermique, une modification moyenne du couple, une modification moyenne de la vitesse de rotation, une émission de suie par le processus de combustion, une émission de NOx créée par le processus de combustion, une vitesse de rotation, un couple, une puissance moyenne, un nombre de démarrages à froid et une durée de séjour en fonctionnement à froid, une qualité de carburant et d'huile.

3. Procédé selon une ou plusieurs des revendications précédentes, **caractérisé en ce que** les indicateurs d'usure comprennent les suivants : une teneur en suie dans l'huile, un indice d'acidité total/indice de basicité total, une viscosité, des corps étrangers dans l'huile, du carburant dans l'huile, une teneur en eau dans l'huile.

4. Moteur à combustion interne, **caractérisée en ce qu'**un procédé selon une ou plusieurs des revendications précédentes est mis en oeuvre.

Zusammensetzung des NRTC Test-Zyklus

| Anwendungs-nummer | Anwendungs-bezeichnung | Dauer der Anwendung gesamt [s] | Anwendungs-bereich | Zeitbereiche der Segmente | Segmentname | Dauer der einzelnen Anwendung [s] | Segmentposition im Zyklus |
|---|---|---|---|---|---|---|---|
| | | | | | Start des transienten Zyklus | 28 | 0-28 |
| 1 | Baggerlader | 206 | 29-234 | 52-86 | Straßenbau | 35 | 29-63 |
| | | | | 108-141 | Schürfen | 34 | 64-97 |
| | | | | 174-218 | Beladen | 45 | 98-142 |
| | | | | 351-442 | Abstufung | 92 | 143-234 |
| 2 | Radlader | 184 | 235-418 | 746-822 | Typische Anwendung | 77 | 235-311 |
| | | | | 531-637 | Transientbetrieb hohe Drehzahl | 107 | 312-418 |
| 3 | Planierraupe | 209 | 419-627 | 85-206 | Straßenbett anlegen | 122 | 419-540 |
| | | | | 376-462 | Räumen | 87 | 540-627 |
| 4 | Traktor | 150 | 628-777 | 265-414 | Landwirtschaftl. Anwendung | 150 | 628-777 |
| 5 | Bagger | 35 | 778-812 | 319-338 | Geringe Leistung (128 PS) | 20 | 778-797 |
| | | | | 431-445 | Höhere Leistung (208 PS) | 15 | 798-812 |
| | | | | | Transition | 3 | 813-815 |
| 6 | Lichtbogenschweißgerät | 204 | 816-1019 | 1007-1103 | Typische Anwendung | 97 | 816-912 |
| | | | | 544-650 | Transientbetrieb hohe Drehzahl | 107 | 913-1019 |
| 7 | Kompaktlader | 185 | 1020-1204 | 264-365 | Typische Anwendung | 102 | 1020-1121 |
| | | | | 150-232 | Transientbetrieb hohes Drehmoment | 83 | 1122-1204 |
| | | | | | Leerlauf und Ende des transienten Zyklus | 34 | 1215-1238 |

**Fig. 1**

Fig. 2

EP 3 891 368 B1

Künstliches Neuronales Netz (A)

Ruß [ppm]

Öltemp.- Integral [°C / 24h]

...

$\sum Ru\beta gehalt\ [\%]$

∂Rußgehalt [%]

Schwellen-überwachung

Forecast Berechnung via exp. Glättung 2.Ordnung

Künstliches Neuronales Netz (B)

Ruß [ppm]

Öltemp.- Integral [°C / 24h]

...

$\sum Viskosität\ [m^2/s^{-1}]$

∂Viskosität [m²/s⁻¹]

Schwellen-überwachung

Forecast Berechnung via exp. Glättung 2.Ordnung

...

...

OR

Warnung

...

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2018202333 A1